# EUROPÄISCHE PATENTSCHRIFT

(11) **EP 2 563 428 B1**
(45) Veröffentlichungstag und Bekanntmachung des Hinweises auf die Patenterteilung: **31.12.2014**
(21) Anmeldenummer: 11716373.3
(22) Anmeldetag: 13.04.2011
(51) Int. Cl.: A61M 5/20, A61M 5/30, A61M 5/31, A61M 5/50

(54) **EINMALINJEKTOR MIT BIEGEELASTISCHEM KOLBENBETÄTIGUNGSSTEMPEL**
SINGLE-USE INJECTOR HAVING A FLEXURALLY ELASTIC PISTON ACTUATING PLUNGER
INJECTEUR À USAGE UNIQUE DOTÉ D'UN POUSSOIR D'ACTIONNEMENT DE PISTON ÉLASTIQUE ET FLEXIBLE

(30) Priorität: 27.04.2010 DE 102010018529
(43) Veröffentlichungstag der Anmeldung: 06.03.2013
(73) Patentinhaber: LTS LOHMANN Therapie-Systeme AG, 56626 Andernach (DE)
(72) Erfinder: MATUSCH, Rudolf, 35041 Marburg (DE)
(86) Internationale Anmeldenummer: PCT/EP2011/001842
(87) Internationale Veröffentlichungsnummer: WO 2011/134601

(56) Entgegenhaltungen:
- EP-A2- 0 114 145
- WO-A1-2006/062997
- WO-A2-2007/002052
- DE-A1-102004 060 146

## Beschreibung

Die Erfindung betrifft einen Einmalinjektor mit einer - zumindest zeitweise wirkstoffbefüllbaren - Zylinder-Kolben Einheit, mit einem Gehäuse, in dem ein vorgespannter und freigebbarer mechanischer Federenergiespeicher angeordnet ist und mit mindestens einem zwischen dem Federenergiespeicher und dem Kolben der Zylinder-Kolben-Einheit positionierten Kolbenbetätigungsstempel, wobei der Kolbenbetätigungsstempel zwei Zughaken aufweist, die das Gehäuse bereichsweise umgreifen und der Kolbenbetätigungsstempel mittels des sich entspannenden Federenergiespeichers vom Gehäuse trennbar ist.

Ein Injektor mit diesen Merkmalen ist z.B. aus der EP 0 114 145 A2 bekannt.

Weiterhin ist aus der DE 10 2007 031 630 A1 ein derartiger Injektor bekannt. Mit Ausnahme der mechanischen Feder des Federenergiespeichers sind nahezu alle Bauteile des Injektors aufwendig aus Kunststoffen durch Spritzgießen gefertigt. Mechanisch hochbelastete Bauteile sind zusätzlich glasfaserverstärkt ausgeführt.

Der vorliegenden Erfindung liegt daher die Problemstellung zugrunde, einen modular aufgebauten Einmalinjektor zu entwickeln, der bei geringer Baugröße nur wenige Bauteile aufweist und bei einfacher Handhabung sowie einer kostengünstigen Herstellung eine sichere Lagerung und Funktion gewährleistet.

Diese Problemstellung wird mit den Merkmalen des Hauptanspruchs gelöst. Dazu sind sowohl das Gehäuse als auch der Kolbenbetätigungsstempel dünnwandige Blechteile.

Weitere Einzelheiten der Erfindung ergeben sich aus den Unteransprüchen und den nachfolgenden Beschreibungen schematisch dargestellter Ausführungsbeispiele.
- Figur 1:: Einmalinjektor;
- Figur 2:: wie Figur 1, jedoch um 90 Winkelgrade geschwenkt;
- Figur 3:: Querschnitt zu Figur 2;
- Figur 4:: Gehäuse mit Kolbenbetätigungsstempel;
- Figur 5:: Einmalinjektor bei einem Montagezwischenschritt;
- Figur 6:: wie Figur 1, jedoch entsichert und betätigt;
- Figur 7:: Einmalinjektor mit alternativer Auslöseeinheit;
- Figur 8:: wie Figur 7, jedoch ausgelöst;
- Figur 9:: Gehäuse mit Kolbenbetätigungsstempel;
- Figur 10:: Detail der Auslöseeinheit aus Figur 8;
- Figur 11:: Zwischenschritt beim Zusammenbau;
- Figur 12:: wie Figur 7, jedoch entsichert und betätigt;
- Figur 13:: Einweginjektor mit Auslöseeinheit für Rohre mit konstantem Innenquerschnitt;
- Figur 14:: Gehäuse und Stempel von Figur 13;
- Figur 15:: Einmalinjektor mit Einsatzgehäuse;
- Figur 16:: wie Figur 15, jedoch um 90 Grad geschwenkt.

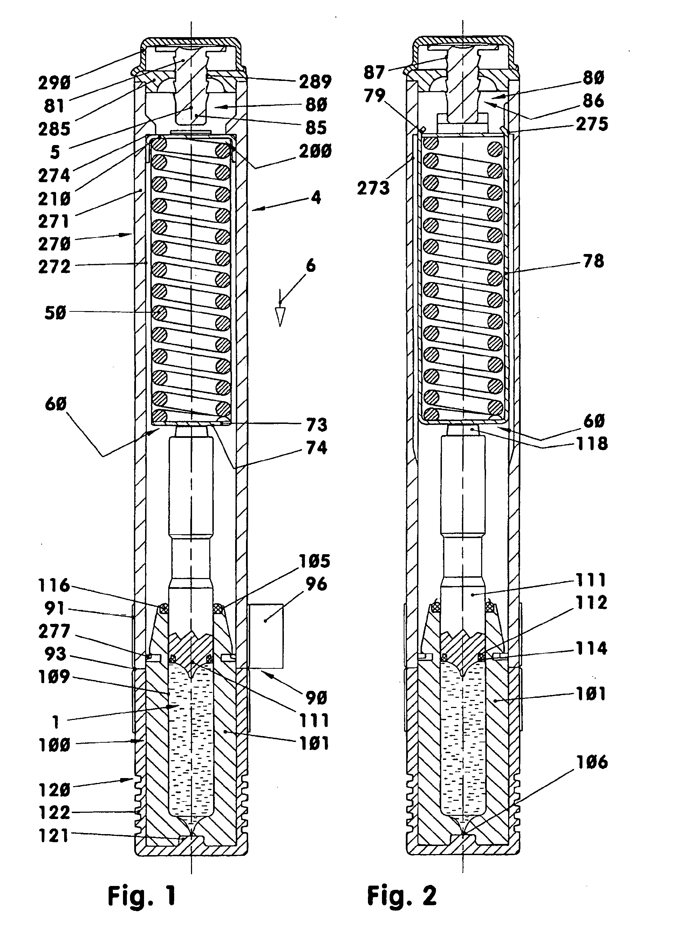

Die Figuren 1 bis 3 zeigen einen Einmal- bzw. Einweginjektor (4) mit einem dauergeladenen Federenergiespeicher (50). Der Einweginjektor (4) umfasst ein Mantelgehäuse (270), in dem ein inneres Gehäuse (200), ein Kolbenbetätigungsstempel (60), eine Schraubendruckfeder als Federenergiespeicher (50) und eine

Zylinder-Kolben-Einheit (100) angeordnet sind. Die Zylinder-Kolben-Einheit (100) sitzt dabei großteils in einer Schutzkappe (120).

Das hülsenförmige Mantelgehäuse (270) umfast ein Rohrteil (271) mit quadratischem Querschnitt und einen Deckel (285). Die beispielsweise weitgehend glatte Umfangsfläche des Rohrteils (271) kann eingeprägte oder aufgeklebte Etiketten, Beschriftungen, etc. aufweisen. Die Innenwandung (272) weist beispielsweise einander gegenüberliegende Freigabenuten (273) aus. Diese Freigabenuten (273) sind im Ausführungsbeispiel halb so lang wie das Rohrteil (271). In der Darstellung der Figur 2 liegt das obere, z.B. scharfkantige Ende der Freigabenuten (273) drei Millimeter unterhalb des oberen Endes des Kolbenbetätigungsstempels (60). Oberhalb der Freigabenuten (273) bildet die Innenwandung Sperrerflächen (275), an denen der Kolbenbetätigungsstempel (60) anliegt.

Der Deckel (285), der an das Vierkantrohr (271) angeformt sein kann, hält in einer z.B. zentralen Bohrung (289) die Auslöseeinheit (80). Diese umfasst einen manuell betätigbaren, beispielsweise pilzförmigen Auslöser (81) mit einer Verliersicherung (86). Im Ausführungsbeispiel wird die Verliersicherung (86) durch an der Umfangsfläche des Auslöserstößels (85) versetzt zueinander angeordnete Rastkeile (87) oder -haken gebildet, die die Innenseite des Deckels (285) oder eine Hintergriffsfläche des Mantelrohrs (271) hintergreifen. Auch Gleitkufen sind anstatt Widerhaken denkbar. Die Auslöseeinheit (80) ist mit einer Schutzkappe (290) abgedeckt. Gegebenenfalls kann eine zusätzliche Verriegelung vorgesehen sein, um ein unbeabsichtigtes Betätigen zu verhindern.

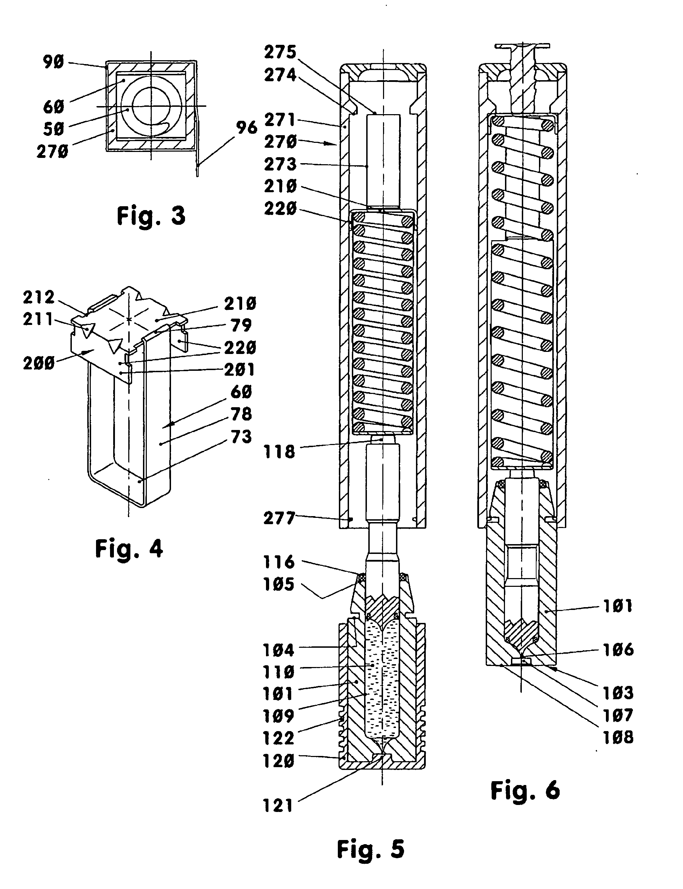

Das Gehäuse (200) ist ein zu einem "U" gebogener Blechstreifen (201), vgl. Figur 4. Der abgewickelte, z.B. 18 Millimeter breite, Blechstreifen (201) ist ca. 50 Millimeter lang. Der ggf. aus Federstahl gefertigte Blechstreifen (201) hat eine Wandstärke von z.B. 0,5 Millimeter. Der gebogene Blechstreifen (201) besteht aus einer zentralen Stirnplatte (210) und zwei davon zumindest annähernd senkrecht abstehenden Schenkeln (220). Er hat keine Bohrungen oder Durchbrüche und kann daher im Wesentlichen mittels einfacher Trenn- und Umformschritte hergestellt werden.

An den Übergangsstellen zwischen der Stirnplatte (210) und den Schenkeln (220) sind nach Figur 4 jeweils zwei Versteifungssicken (211) eingedrückt. Die Versteifungssicken (211) ragen soweit in die Stirnplatte (210) hinein, dass sie zudem die letzte Windung der Schraubendruckfeder (50) auf der Stirnplatte (210) zentrieren.

Der Kolbenbetätigungsstempel (60) ist ein u-förmig gebogener Blechstreifen, der aus einem Mittelteil, dem Stempelteller (73) und zwei Führungsschenkeln (78) besteht. Im Ausführungsbeispiel hat er keine Bohrungen oder Durchbrüche. Er ist daher mittels kostengünstiger Biege- und/oder Abkantvorgänge herstellbar. Der Stempelteller (73) ist parallel zur Stirnplatte (210) orientiert. Die biegeelastischen Führungsschenkel (78) stehen rechtwinkelig nach oben ab. Zwischen den Führungsschenkeln (78) sitzt die Schraubendruckfeder (50). Ggf. sind die Führungsschenkel (78) gegenüber dem Stempelteller (73) mit Versteifungssicken versteift, vgl. hierzu die Versteifungssicken (211) des Blechteils (201).

An ihrem oberen Ende haben die beiden Führungsschenkel (78) abgewinkelte Umgriffe (79), vgl. die Figuren 2 und 4. Diese schließen beispielsweise mit den Führungsschenkeln (78) einen Winkel von 135 Grad ein. Der eingeschlossene Winkel kann beispielsweise zwischen 100 Grad und 170 Grad liegen. Die Umgriffe (79) umgreifen bzw. hintergreifen die Stirnplatte (210) bereichsweise, so dass in der Darstellung der Figuren 2 und 4 der Kolbenbetätigungsstempel (60) am Gehäuse (200) hängt.

Gemäß Figur 1 hat der Kolbenbetätigungsstempel (60) eine Breite, die geringfügig - also ca. 0,1 bis 0,3 Millimeter - kleiner ist als der reguläre Abstand der beiden Schenkel (220). In Figur 2 ist zu erkennen, dass die Führungsschenkel (78) mit den Haken (79) des Kolbenbetätigungsstempels (60) nur die oberen drei Millimeter an der Innenwandung (272) bzw. den Sperrerflächen (275) des Mantelgehäuses (270) anliegen. Sobald diese Strecke beim Auslösen überwunden ist, springen die Haken (79) in die Freigabenuten (273), so dass die Feder (50) den Stempelteller (73) vorantreibt.

Der Stempelteller (73) kann eine zentrale Bohrung aufweisen, um den dann mit einem Zapfen versehenen Kolben (111) der Zylinder-Kolben-Einheit (100) rückwärtig zusätzlich zu führen.

Das aus Blech gefertigte Gehäuse (200) ist großteils vom Mantelgehäuse (270) umgeben, in dem es gleitfähig sitzt. Das aus Metall, z.B. Aluminium oder Kunststoff, z.B. einem Polyamid gefertigte Mantelrohr (271) hat beispielsweise eine Wandstärke von 1,5 bis 2,5 Millimetern. In seinem hinteren Bereich weist es einen Montageanschlag (274) auf.

Die Zylinder-Kolben-Einheit (100) besteht im Ausführungsbeispiel aus einem, mit einer Injektionslösung (1) oder einem Lösemittel, z.B. Wasser für Injektionszwecke, befüllten, transparenten Zylinder (101), in dem nach den Figuren 1 und 2 der Kolben (111) in seiner hinteren Position sitzt.

Der Zylinder (101) ist z.B. ein dickwandiger Topf. Die Zylinderbohrung ist beispielsweise zylindrisch oder kegelstumpfmantelförmig ausgeführt. Im Zentrum der Bohrung, deren Zylinderboden der Kontur der vorderen Stirnseite des Kolbens (111) zumindest annähernd angepasst ist, befindet sich z.B. eine kurze zylindrische, düsenartige Bohrung (106). Ihr Durchmesser beträgt ca. 0,1 bis 0,5 Millimeter. Diese Bohrung (106) ist ein- bis fünfmal so lang wie ihr Durchmesser. Sie endet in einer zylindrischen Ausnehmung (107) der bodenseitigen, äußeren Stirnfläche (103) des Zylinders (101), vgl. Figur 6. Ggf. können im Boden des Zylinders (101) auch zwei oder mehr düsenartige Bohrungen (106) angeordnet sein.

Um die Ausnehmung (107) herum klebt fest haftend auf der Stirnfläche (103) ein Klebering (108). Letzterer deckt nahezu die gesamte Stirnfläche (103) ab.

Die räumliche Außenkontur des Zylinders (101) ist im Ausführungsbeispiel z.B. quaderförmig gestaltet. Sie kann jedoch auch zylindrisch sein. Der Querschnitt der Außenkontur - er ist quer zur Mittellinie (5) orientiert - ist im mittleren Zylinderbe-reich eine quadratische Fläche mit zentraler Bohrung.

Der Zylinder (101) hat in seiner Außenkontur im oberen, dem Vierkantrohr (270) zugewandten Viertel eine z.B. umlaufende Haltekerbe (104) mit einem beispielsweise rechteckigen Kerbquerschnitt. Oberhalb der Haltekerbe (104) verjüngt sich der Zylinder (101) pyramidenstumpfförmig. Der von gegenüberliegenden pyramidalen Flächen eingeschlossene Winkel beträgt z.B. 20 bis 30 Winkelgrade. Die Haltekerbe (104) kann ggf. auch nur aus zwei einander gegenüber liegenden Einzelkerben bestehen.

Der Zylinder (101) hat eine Zylinderinnenwandung (109), die im Bereich der hinteren Zylinderstirnfläche in einer Ringnut (105) zur Aufnahme eines Dichtelements (116) endet.

Der im Zylinder (101) sitzende Kolben (111) hat an seiner vorderen, zumindest annähernd kegelig gestalteten Stirnfläche eine axiale Ringnut (112) zur Aufnahme eines Dichtringes (114) oder einer dauerelastischen Dichtmasse. Der Kolben (111) hat in seinem mittleren Bereich eine Taille und an seiner Rückseite z.B. einen zentralen, kegelstumpfförmigen Zapfen (118). Der Kolben (111) und das Dichtelement (116) sowie den Stopfen (121) schließen den befüllten Zylinderinnenraum (110) steril ab.

In den Darstellungen der Figuren 1 und 2 ist eine topfförmige Schutzkappe (120) von unten her auf den Zylinder (101) aufgesteckt. Die einteilige Schutzkappe (120), die geometrisch im Prinzip aus fünf ebenen Wandungen besteht, umschließt den Zylinder (101) seitlich mit geringem Spiel. Ihre obere, z.B. plane Stirnfläche kontaktiert die vordere Stirnfläche des vierkantförmigen Mantelgehäuses (270). Die Außenwandung der Schutzkappe (120) weist eine Profilierung oder Struktur auf, um das Abziehen vom Zylinder (101) zu erleichtern. Im Ausführungsbeispiel wird als Profilierung ein Rillenprofil (122) verwendet.

Der Boden der Schutzkappe (120) weist einen Stopfen (121) auf, der dichtend in die Ausnehmung (107) des Zylinders (101) hineinragt, vgl. die Figuren 1 und 6. Die Schutzkappe (120) haftet am Zylinder (101) über den Klebering (108). Letzterer hat gegenüber dem Zylinder (101) eine wesentlich höhere Haftkraft als gegenüber dem Boden der Schutzkappe (120). Um die Haftkraftdifferenz zusätzlich sicherzustellen, ist ggf. der Boden mit einem Profil oder Absatz versehen, so dass die Kontaktfläche gegenüber dem Klebering (108) kleiner ist als die Kontaktfläche zwischen dem Klebering (108) und der zylinderseitigen Stirnfläche (103).

Zwischen dem Stempelteller (73) und der Stirnplatte (210) des Blechstreifens (201) sitzt vorgespannt die Schraubendruckfeder (50). Die Federkraft - im gespannten Zustand beträgt sie beispielsweise 500 Newton - wird über den Stempelteller (73) auf die die Stirnplatte (210) hintergreifenden Zughaken (78) übertragen.

Nach den Figuren 1 und 2 ist der Berührbereich des vierkantförmigen Mantelrohrs (271) und der Schutzkappe (120) mit einem Originalitätsverschluss, z.B. einer Banderole (90) als Sicherungselement, verschlossen. Die abreiß- oder auftrennbare Banderole (90) ist z.B. ein mit einem Klebstoff einseitig beschichteter Papier- oder Folienstreifen. Der Folienstreifen umgibt z.B. einlagig einmal den Verbund aus Mantelgehäuse (270) und Schutzkappe (120). Er verklebt die Teile (270) und (120) temporär. Zum Entsichern des Injektors bzw. zum Entfernen der Schutzkappe (120) - bei der Vorbereitung zur Nutzung des Injektors - wird die Banderole (90) abgezogen oder so aufgetrennt, dass die Klebeverbindung zwischen dem Mantelrohr (270) und der Schutzkappe (120) aufgehoben ist. Im Ausführungsbeispiel wird dazu die im Bereich des Mantelgehäuses (270) liegende Abreißfahne (96) ergriffen und damit die Banderole (90) z.B. bereichsweise abgewickelt. Hierbei reißt die Banderole (90) an einer definierten, z.B. geradlinigen Sollbruchstelle (93) auf, die genau im Bereich der Stirnseiten liegt. Folglich wird - beim Entsichern - nur der auf dem Mantelgehäuse (270) anliegende Teil (91) der Banderole (90) entfernt.

Die Figur 5 zeigt den Injektor (4) bei einem Montagezwischenschritt. Bei der Montage wird zunächst die Schraubendruckfeder (50) mit dem Kolbenbetätigungsstempel (60) und dem Blechstreifen (201) zusammengesteckt. Dazu wird die Schraubendruckfeder (50) in den fertig umgeformten Blechstreifen (201) so eingelegt, dass ein Federende an der Stirnplatte (210) zur Anlage kommt. Auf das andere Federende wird der bügelartige Kolbenbetätigungsstempel (60) aufgeschoben. Nun wird unter Zuhilfenahme einer die Schraubendruckfeder (50) außen oder innen führenden Montagevorrichtung der Blechstreifen (201) zwischen der Stirnplatte (210) und dem Kolbenbetätigungsstempel (60) so weit - entgegen der Federwirkung - zusammengeschoben, dass die Umgriffe (79) an der Stirnplatte (210) einrasten.

Nun wird die Kombination aus der gespannten Feder (50), dem Blechstreifen (201) und dem Kolbenbetätigungsstempel (60) - immer noch eingespannt in der Montagevorrichtung - von unten her in das Mantelgehäuse (270) eingeschoben.

Die Stirnplatte (210) wird bis zum Montageanschlag (274) vorgeschoben. Die Haken (79) sind dann durch die Sperrerflächen (275) blockiert. Nun kann das Montagewerkzeug entnommen werden. Die Feder (50) ist blockiert und kann nicht auslösen.

In einem weiteren Montageschritt wird die befüllte Zylinder-Kolben-Einheit (100), mit dem Führungszapfen (118) des Kolbens (111) voraus, in das Vierkantrohr (270) eingesteckt. Die Rastelemente (277) des Mantelgehäuses (270) greifen fest in die Haltekerbe (104) ein und fixieren so die Zylinder-Kolben-Einheit (100) im Vierkantrohr (270). Die an dem Kolbenbetätigungsstempel (60) anliegenden Sperrerflächen (275) sichern dessen Lage stabil. Zu dem in Figur 1 dargestellten Montageschritt fehlt nur noch das Anbringen des Originalitätsverschlusses (90).

Zur Vorbereitung der Benutzung des in den Figuren 1 bis 6 dargestellten Einweginjektors wird zunächst die Abreißfahne (96) und der hintere Banderolenabschnitt (91) abgelöst. Anschließend wird die Schutzkappe (120) von der Zylinder-Kolben-Einheit (100) abgezogen. Nun wird der Injektor mit dem Klebering (108) voraus auf der Injektionsstelle positioniert. Dabei wird der Einweginjektor (4) am Vierkantrohr (270) in der Faust gehalten. Der Daumen der haltenden Hand liegt beispielsweise auf dem Auslöser (81) auf, z.B. wie beim Halten eines Kugelschreibers.

Nun wird das Auslöseelement (81) in Richtung der Zylinder-Kolben-Einheit (100) verschoben. Der Auslösestößel (85) verschiebt das Gehäuse (200) relativ zum Mantelgehäuse (270). Gegebenenfalls rastet das Auslöseelement (81) mit weiteren Rastkeile (87) in den Deckel (285) ein. Die Führungsschenkel (78) des Kolbenbetätigungsstempels (60) gleiten - unter Verringerung ihrer Anlagefläche - an den Sperrerflächen (275) entlang nach unten. Sobald die Führungsschenkel (78) nicht mehr an den Sperrerflächen (275) anliegen, ist die stabile Sicherung gelöst. Die sich am Gehäuse (200) abstützende und auf den Stempelteller (73) wirkende Feder (50) drückt den Kolbenbetätigungsstempel (60) nach unten. Hierbei springen die Umgriffe (79) aus der Verrastung mit der Stirnplatte (210) nach außen in die Freigabenuten (273). Der Kolbenbetätigungsstempel (60) wird vom Gehäuse (200) gelöst und freigegeben. Das Gehäuse (200) wird beispielsweise gegen den Montageanschlag (274) zurückgeschleudert. Die Stirnseite (74) des Stempeltellers (73) schlägt auf die Stirnseite des bisher etwa drei Millimeter entfernt gelegenen Kolbens (111). Der Kolben (111) drückt die Injektionslösung bzw. das Medikament (1) z.B. anfangs mit 200 x 10⁵ Pa durch die Düse (106), bis der Zylinder (101) entleert ist, vgl. Figur 6. Mit der Abgabe der Injektionslösung (1) ist der Injektionsvorgang beendet.

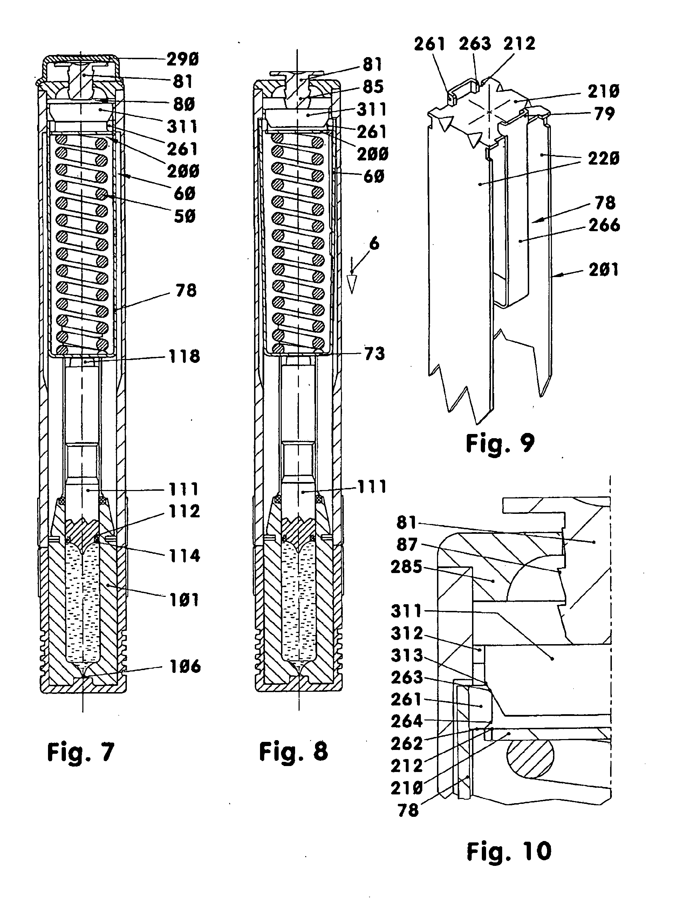

Die Figuren 7 - 11 zeigen einen Einmalinjektor (4), der sich im Aufbau der Auslöseeinheit (80) und im Aufbau des Gehäuses (200) von dem vorstehend beschriebenen Injektor unterscheidet. Die Zylinder-Kolben-Einheit (100) ist so aufgebaut wie im Zusammenhang mit dem ersten Ausführungsbeispiel beschrieben.

Das Gehäuse (200) weist zwei lange und breite Schenkel (220) auf, deren freie Enden jeweils z.B. um einen Winkel von 90 Grad abgewinkelt sind. Sie bilden dort ein beispielsweise zwischen 1,5 und drei Millimeter lange, auf einander zu ragende Halteelemente (221), die in einer Ebene parallel zur Stirnplatte (210) ausgerichtet sind. Anstelle des hakenförmigen Halteelements (221) kann in jedem Schenkel (220) eine Ausnehmung vorgesehen sein, in die der Zylinder der Zylinder-Kolben-Einheit (100) mittels jeweils eines Zapfens eingehängt werden kann.

Der Kolbenbetätigungsstempel (60) ist ähnlich aufgebaut wie der im Zusammenhang mit dem ersten Ausführungsbeispiel beschriebene Kolbenbetätigungsstempel (60). Die in den Figuren 7 - 9 nach oben über das Gehäuse (200) überstehenden Haken (79) umfassen beidseitig abgebogene Laschen (261). Jede dieser Laschen (261) hat eine die Stirnplatte (210) hintergreifende Verrastungsfläche (262) und eine in Richtung des Auslösers (81) orientierte Führungsfläche (263), vgl. Figur 10. Im Ausführungsbeispiel schließen die Ebenen beider Flächen (262, 263) einen Winkel von 60 Grad ein. Die einzelne Verrastungsfläche (262) und der zugehörige lange Schenkel (266) des Zughakens (78) stehen z.B. im rechten Winkel zueinander.

An jede der Verrastungsflächen (262) schließt eine den langen Schenkeln (266) der Führungsschenkel (78) abgewandte Gleitfläche (264) an. Die beiden genannten Flächen (262, 264) schließen im Ausführungsbeispiel einen Winkel von 135 Grad ein.

Die Auslöseeinheit (80) umfasst ein Auslöseelement (81) und eine Auslösescheibe (311). Das z.B. pilzförmig ausgebildete Auslöseelement (81) ragt mit dem Auslöserstößel (85) in den Deckel (285). Die den Deckel (285) hintergreifenden Rasthaken (87) verhindern hierbei, dass das Auslöseelement (81) aus dem Deckel (285) herausfällt.

Die Auslösescheibe (311) liegt im Ausführungsbeispiel im Mantelgehäuse (270) lose oberhalb des Gehäuses (200). An ihrem Umfang ist sie beispielsweise mittels vier Zentriernasen (312) an der Innenwandung (272) des Vierkantrohrs (270) zentriert. Nach der Darstellung der Figuren 10 hat die Auslösescheibe (311) Einführflächen (313), die zumindest bei betätigter Auslöseeinheit (80) an den Führungsflächen (263) des Kolbenbetätigungsstempels (60) anliegen.

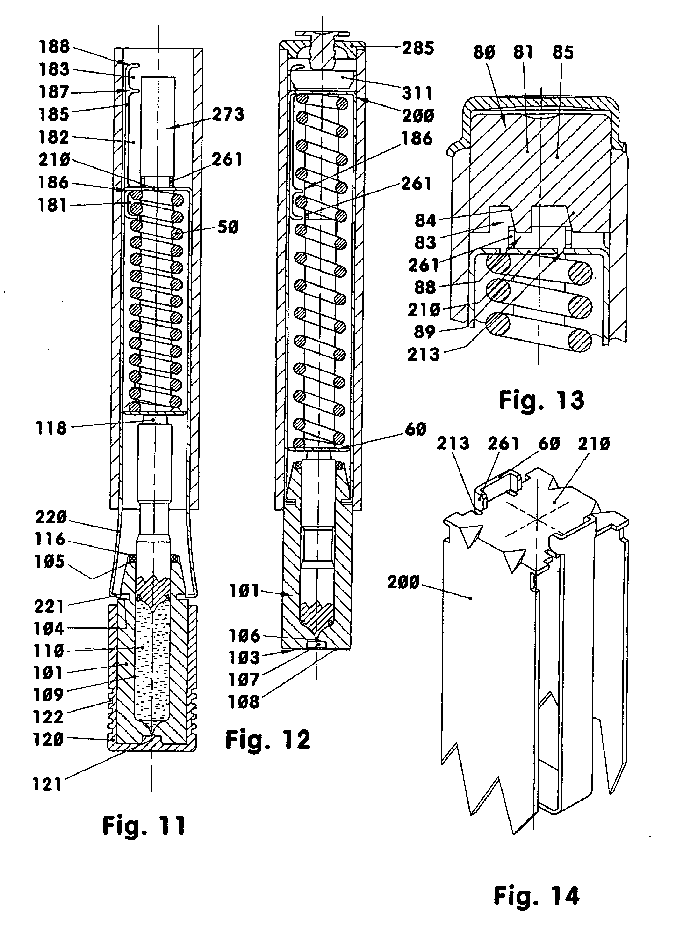

Im hinteren Bereich des Vierkantrohres (270) sind drei wenige zehntel Millimeter nach innen ragende, elastische Rastlaschen (181 - 183) angeordnet, vgl. die Figuren 11 und 12. Die Rastlaschen (181 - 183) haben z.B. jeweils eine rechteckige Form. Ihre Wandstärke entspricht ca. 50% der Wandstärke des Vierkantrohres (270). Sie grenzen sich an drei Seiten gegenüber der Wandung des Vierkantrohres (270) bzw. gegenüber der nächstgelegenen Rastlasche über Spalte (185) ab. Die Spalte (185) haben eine Breite von z.B. 0,5 Millimeter. Die Breite entspricht der Wandstärke der Stirnplatte (210). An den Stellen, an denen jeweils zwei Spalten (185) rechtwinkelig aneinanderstoßen, sind die Rastlaschen (181 - 183) abgerundet.

Die außermittig angeordneten, am Vierkantrohr angeformten Rastlaschen (181 - 183) sichern die Position des Blechstreifens (201) an drei Stellen (186 - 188). Sie ragen dazu mehrere zehntel Millimeter in den Innenraum des Auslöseelements (81) hinein. Die erste Stelle (186) ist der Spalt zwischen der vorderen (181) und der mittleren Rastlasche (182). Im dortigen horizontalen Spalt ist die Stirnplatte (210) eingerastet, vgl. Figur 11, wenn der Blechstreifen (201) mit der zwischen dem Kolbenbetätigungsstempel (60) und der Stirnplatte (210) eingespannten Schraubendruckfeder (50) zur weiteren Zwischenlagerung montiert ist.

Die zweite Stelle (187) ist der Spalt zwischen der mittleren (182) und der hinteren Rastlasche (183). Nach den Figuren 7 und 12 sitzt hier die Stirnplatte (210) bei einem fertig montierten Einweginjektor vor und nach dem Auslösen. Durch das Einrasten der Stirnplatte (210) in diesen Spalt wird ein Herausziehen des Gehäuses (200) aus dem Vierkantrohr (270) - nach dem Abziehen der Schutzkappe (120) - verhindert. Die dritte Stelle (188) ist der Spalt oberhalb der hinteren Rastlasche (183).

Ggf. sind die jeweils oberen Ecken der Rastlaschen (181 - 183) - also die, die dem Deckel (285) zugewandt sind - scharfkantig ausgebildet, so dass der Blechstreifen (201) nur in das Vierkantrohr (270) hineingeschoben werden kann. Eine Bewegung in die entgegengesetzte Richtung ist dann unmöglich.

Die Figur 11 zeigt den Injektor bei einem Montagezwischenschritt. Analog zum ersten Ausführungsbeispiel wird zunächst das Gehäuse (200) mit der Feder (50) und dem Kolbenbetätigungsstempel (60) zusammengebaut.

Nun wird die Kombination aus der gespannten Feder (50), dem Blechstreifen (201) und dem Kolbenbetätigungsstempel (60) - immer noch eingespannt in der Montagevorrichtung - von unten her in das Vierkantrohr (270) eingeschoben. Der Einschiebevorgang ist zunächst beendet, wenn die Stirnplatte (210) in den zwischen den Rastlaschen (181) und (182) gelegenen Spalt (186) einrastet. In dieser Position (186), vgl. Figur 11, ragen die freien Enden der Schenkel (220) unten aus dem Vierkantrohr (270) heraus.

In einem weiteren Montageschritt wird die befüllte Zylinder-Kolben-Einheit (100), mit dem Führungszapfen (118) des Kolbens (111) voraus, in das Vierkantrohr (270) so eingesteckt, dass die Halteelemente (221) der Schenkel (220) in die Haltekerbe (104) des Zylinders (101) hineingreifen. Ausgehend von dieser Position wird das Vierkantrohr (270) weiter über den Blechstreifen (201) geschoben, bis die Stirnplatte (210) in den zwischen den Rastlaschen (182) und (183) gelegenen Spalt (187) einrastet. Hierbei greifen die Halteelemente (221) fest in die Haltekerbe (104) ein und fixieren so die Zylinder-Kolben-Einheit (100) im Vierkantrohr (270). Abschließend wird der Originalitätsverschluss angebracht.

Das Gehäuse (200) kann ohne Schenkel (220) und/oder ohne Halteelemente (221) ausgeführt sein. Die Montage erfolgt dann wie im Zusammenhang mit dem ersten Ausführungsbeispiel beschrieben. Der Stempelteller (73) des Kolbenbetätigungsstempels (60) kann eine zentrale Bohrung zur Aufnahme des Führungszapfens (118) des Kolbens (111) aufweisen.

Zum Auslösen des Einmalinjektors (4) wird nach dem Entfernen des Originalitätsverschlusses (90) und dem Abnehmen der Schutzkappen (120) und (290) der Auslöser (81) betätigt, vgl. die Figuren 8 und 10. Der Auslöserstößel (85) drückt auf die Auslösescheibe (311), die mit ihren Einführflächen (313) auf die Führungsflächen (263) wirkt. Die Führungsflächen (263) werden auseinandergedrückt, bis die Gleitflächen (264) die Kanten (212) der Stirnplatte (210) erreichen.

Die auf die Stirnplatte (210) und den Stempelteller (73) wirkende Schraubendruckfeder (50) zieht die Laschen (261) von der Stirnplatte (210) ab und trennt somit das Gehäuse (200) vom Kolbenbetätigungsstempel (60). Der Kolbenbetätigungsstempel (60) schiebt den Kolben (111) in Richtung der Düse (106), wobei die Injektionslösung (1) durch die Düse (106) ausgestoßen wird.

Die Figuren 13 und 14 zeigen ausschnittsweise einen Einmalinjektor (4), dessen Auslöseeinheit (80) ein Keilgetriebe (83) umfasst. Der Aufbau des Einweginjektors (4) entspricht weitgehend dem Aufbau des im vorhergehenden Ausführungsbeispiel beschriebenen Injektors (4). Die Innenwandung (272) des Mantelgehäuses (270) weist jedoch keine Freigabennuten (273) auf. Im Ausführungsbeispiel hat das Vierkantrohr (270) über seine Länge einen konstanten Innenquerschnitt, wobei alle Innenflächen zueinander identisch sind. Der Innenquerschnitt kann auch eine kreisförmige, achteckige, ovale, etc. Querschnittsfläche haben. Dieser Einweginjektor (4) hat keinen Deckel (285).

Die Stirnplatte (210), die von Rastlaschen (181 - 183) und Spalten (185), vgl. Figur 11, gehalten werden kann, hat im Ausführungsbeispiel vier außermittige Schlitze (213). Zur besseren Darstellung zeigt die Figur 13 hierfür einen Teilausbruch der Stirnplatte (210). Die Länge und Breite des einzelnen Schlitzes (213) ist größer als die entsprechenden Abmessungen einer einzelnen Lasche (261). Der Kolbenbetätigungsstempel (60) ist in der Darstellung der Figuren 13 und 14 so aufgebaut wie der in den Figuren 7 - 12 dargestellte Kolbenbetätigungsstempel (60).

Die Auslöseeinheit (80) umfasst einen z.B. im Mantelgehäuse (270) geführten Auslöser (81) mit einem Auslöserstößel (85). Letzterer hat z.B. eine zumindest bereichsweise quadratische Querschnittsfläche, die zugleich die maximale Querschnittsfläche des Auslöseelements (81) darstellt. An seiner dem Gehäuse (200) zugewandten Stirnfläche (88) trägt der Auslöserstößel (85) zwei Keile (89). Die beispielsweise einen Winkel von 15 Grad zur Längsrichtung (5) einschließenden Keilflächen (84) liegen an den Laschen (261) des Kolbenbetätigungsstempels (60) an.

Beim Betätigen des Auslöseelements (81) werden die Laschen (261) mittels der Keilgetriebe (83) in der Darstellung der Figur 13 nach links verschoben. Aufgrund des kleinen Keilwinkels muß der Bediener hierfür nur eine geringe Kraft aufbringen. Sobald die Laschen (261) über den Schlitzen (213) stehen, zieht die sich entspannende Feder (50) die Laschen (261) durch die Aussparungen (213) hindurch nach unten. Der Kolbenbetätigungsstempel (60) wird vom Gehäuse (200) getrennt und schiebt den Kolben (111) in Richtung der Düse (106). Wegen der langen Führung des nicht auskragenden Auslöseelements (81) besteht keine Kippgefahr und keine Gefahr, dass der Bediener einen Finger einklemmen könnte. Im Ausführungsbeispiel bleibt während des Auslösens und nach dem Auslösen die Größe der durch die Hüllkonturen der Stirnplatte (210) und der Zughaken (78) begrenzte Querschnittsfläche konstant. Der Flächeninhalt der genannten Fläche kann jedoch nach dem Auslösen - ja nach Vorspannung der biegeelastischen Führungsschenkel (78) auch verringert werden.

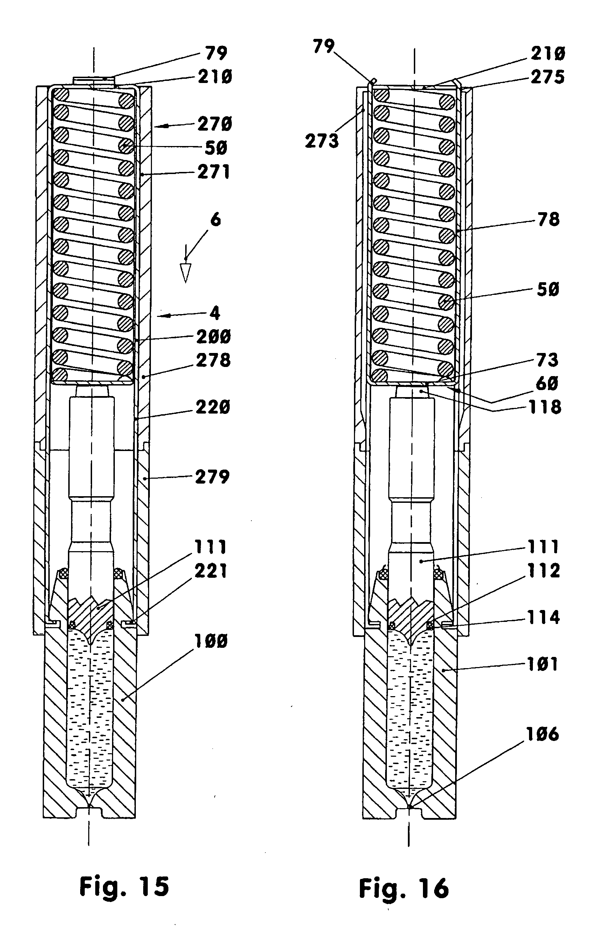

Die Figuren 15 und 16 zeigen einen weiteren Einmalinjektor (4) mit einem Einsatzgehäuse. Der Kolbenbetätigungsstempel (60) und die Zylinder-Kolben-Einheit (100) sind so aufgebaut wie im Zusammenhang mit den Figuren 1 - 6 beschrieben. Das Gehäuse (200) hat im Gegensatz zu dem dort dargestellten Gehäuse (200) lange Schenkel (220) und Halteelemente (221). Diese sind so ausgeführt, wie im Zusammenhang mit den Figuren 7 - 12 beschrieben. Der Einweginjektor (4) hat kein separates Auslöseelement. Das Vierkantrohr (270) ist zweiteilig ausgeführt. Die Länge des oberen Teils (278) beträgt in diesem Ausführungsbeispiel etwa die Hälfte der Länge der in den Figuren 1 - 12 dargestellten Rohrteils (271). Wie in diesen Ausführungsbeispielen hat das Mantelgehäuse (270) Sperrerflächen (275) und Freigabenuten (273). Die Sperrerflächen (275) sind hier z.B. zwei Millimeter hoch. In den Darstellungen der Figuren 15 und 16 ist die Schutzkappe (120) abgenommen.

Bei der Montage wird das Gehäuse (200) mit der Feder (50) und dem Kolbenbetätigungsstempel (60) so weit in das Vierkantrohr (270) eingesetzt, bis die Stirnplatte (210) etwa 0,5 Millimeter aus dem Mantelrohr (270) hervorsteht. Die Haken (79) stehen dann etwa einen Millimeter heraus. Nach dem Einsetzen der Zylinder-Kolben-Einheit (100) wird die untere Hälfte (279) des Mantelrohrs (271) aufgeschoben und fixiert. Hiermit werden die Halteelemente (221) gesichert. Dann wird die Schutzkappe (120) aufgesetzt. Nach Anbringen des hier nicht dargestellten Originalitätsverschlusses ist der Injektor (4) fertig konfektioniert.

Vor dem Einsatz des Einmalinjektors (4) wird der Originalitätsverschluss und die Schutzkappe (120) abgenommen. Nun wird der Injektor (4) wie ein Kugelschreiber in die Hand genommen und auf die Injektionsstelle aufgesetzt. Zum Auslösen drückt der Bediener mit dem Daumen die Stirnplatte (210) etwa zwei Millimeter tief ein. Das Gehäuse (200) mit dem daran hängenden Kolbenbetätigungsstempel (60) wird relativ zum Mantelrohr (270) in den Darstellungen der Figuren 15 und 16 nach unten geschoben. Hierbei verlassen unter der Wirkung der Feder (50) die Haken (79) die Sperrerflächen (275) und springen in die Freigabenuten (273). Die Feder (50) beschleunigt den Kolbenbetätigungsstempel (60) in der Auslösebewegungsrichtung (6). Beispielsweise nachdem dieser sich vom Gehäuse (200) gelöst hat, trifft er auf den zunächst vom Stempelteller (73) beabstandeten Kolben (111). Letzterer wird in Richtung der Düse (106) getrieben, wobei die Injektionslösung (1) ausgespritzt wird.

Der in den letzgenannten Figuren 15 und 16 dargestellte Einmalinjektor (4) besteht nur aus acht Bauteilen. Diese Teile sind das Gehäuse (200), der Kolbenbetätigungsstempel (60), die Feder (50), zwei Viekantrohrabschnitte (278, 279), ein Zylinder (101), ein Kolben (111) sowie eine Schutzkappe (120). Auch Kombinationen der verschiedenen Ausführungsbeispiele sind denkbar.

### Bezugszeichenliste:

- 1: Injektionslösung; Medikament
- 4: Einmalinjektor, Einweginjektor
- 5: Mittellinie des Injektors, Längsrichtung
- 6: Auslösebewegungsrichtung von (81), Abwärtsbewegung Richtungspfeil

- 50: Federelement, Schraubendruckfeder, Federenergiespeicher

- 60: Kolbenbetätigungsstempel

- 73: Stempelteller
- 74: Stirnseite, unten

- 78: Führungsschenkel, Zughaken
- 79: Umgriffe, Haken

- 80: Auslöseeinheit
- 81: Auslöser, Auslöseelement

- 83: Keilgetriebe
- 84: Keilflächen
- 85: Auslöserstößel
- 86: Verliersicherung
- 87: Rastkeile, Gleitkufen, Rasthaken
- 88: Stirnfläche
- 89: Keile

- 90: Originalitätsverschluss, Banderole, Sicherungselement
- 91: hinterer Banderolenabschnitt, an (270); Teil
- 93: Sollbruchstelle, Perforation
- 96: Abreißfahne

- 100: Zylinder-Kolben-Einheit
- 101: Zylinder
- 103: Stirnfläche
- 104: Haltekerbe
- 105: Ringnut
- 106: Bohrung, Düse
- 107: Ausnehmung in der Stirnfläche
- 108: Klebering
- 109: Zylinderinnenwandung
- 110: Zylinderinnenraum
- 111: Kolben
- 112: Ringnut
- 114: Dichtring, Dichtung
- 116: Dichtelement in (105)
- 118: Führungszapfen

- 120: Schutzkappe
- 121: Stopfen
- 122: Rillenprofil

- 181: Rastlasche, vorn; Rastelement
- 182: Rastlasche, Mitte; Rastelement
- 183: Rastlasche, hinten; Rastelement

- 185: Spalte
- 186: 1. Stelle
- 187: 2. Stelle
- 188: 3. Stelle

- 200: Gehäuse; Blechteil, dünnwandig
- 201: Blechstreifen; Blechteil

- 210: Stirnplatte
- 211: Sicken, Versteifungssicken
- 212: Kanten
- 213: Schlitze, Aussparungen

- 220: Schenkel
- 221: Halteelemente

- 261: Laschen
- 262: Verrastungsfläche
- 263: Führungsfläche
- 264: Gleitfläche

- 266: lange Schenkel von (78)

- 270: Mantelgehäuse, Vierkantrohr
- 271: Rohrteil, Mantelrohr
- 272: Innenwandung
- 273: Freigabenuten
- 274: Montageanschlag
- 275: Sperrerflächen
- 277: Rastelemente
- 278: oberer teil von (271)
- 279: unterer teil von (271)

- 285: Deckel
- 289: Bohrung

- 290: Schutzkappe

- 311: Auslösescheibe
- 312: Zentriernasen
- 313: Einführflächen

## Patentansprüche

1. Einmalinjektor (4) mit einer - zumindest zeitweise wirkstoffbefüllbaren - Zylinder-Kolben-Einheit (100), mit einem Gehäuse (200), in dem ein vorgespannter und freigebbarer mechanischer Federenergiespeicher (50) angeordnet ist und mit mindestens einem zwischen dem Federenergiespeicher (50) und dem Kolben (111) der Zylinder-Kolben-Einheit (100) positionierten Kolbenbetätigungsstempel (60), wobei
- der Kolbenbetätigungsstempel (60) zwei Zughaken (78) aufweist, die das Gehäuse (200) bereichsweise umgreifen,
- der Kolbenbetätigungsstempel (60) mittels des sich entspannenden Federenergiespeichers (50) vom Gehäuse (200) trennbar ist, **dadurch gekennzeichnet,**
**dass** sowohl das Gehäuse (200) als auch der Kolbenbetätigungsstempel (60) dünnwandige Blechteile sind.

2. Einmalinjektor (4) nach Anspruch 1,
**dadurch gekennzeichnet,**
**dass** zumindest das Gehäuse (200) eine Stirnplatte (210) mit Schlitzen (213) umfasst.

3. Einmalinjektor (4) nach Anspruch 1,
**dadurch gekennzeichnet,**
**dass** das Gehäuse (200) von einem Mantelgehäuse (270) mit einem über seine Länge konstanten Innenquerschnitt umgeben ist.

4. Einmalinjektor (4) nach Anspruch 1,
**dadurch gekennzeichnet,**
**dass** die normal zur Längsrichtung (5) orientierte maximale Querschnittsfläche des Gehäuses (200) und des Kolbenbetätigungsstempels (60) der Hüllkonturen der Flächen der Stirnplatte (210) und der sie bereichsweise umgreifenden Führungsschenkel (78) entspricht.

5. Einmalinjektor (4) nach Anspruch1,
**dadurch gekennzeichnet,**
**dass** die Führungsschenkel (78) jeweils einen abgewinkelten Umgriff (79) aufweisen.

## Claims

1. Disposable injector (4) with a cylinder/piston unit (100) that can be filled at least temporarily with active substance, with a housing (200) in which a pretensioned and releasable mechanical spring energy reservoir (50) is arranged, and with at least one piston-actuating ram (60) positioned between the spring energy reservoir (50) and the piston (111) of the cylinder/piston unit (100), wherein
- the piston-actuating ram (60) has two draw hooks (78) that engage around the housing (200) in some areas,
- the piston-actuating ram (60) is separable from the housing (200) by means of the relaxing spring energy reservoir (50), **characterized in that** both the housing (200) and also the piston-actuating ram (60) are thin-walled sheet-metal parts.

2. Disposable injector (4) according to Claim 1, **characterized in that** at least the housing (200) comprises an end plate (210) with slits (213).

3. Disposable injector (4) according to Claim 1, **characterized in that** the housing (200) is surrounded by a jacket housing (270) that has a constant inner cross section along its length.

4. Disposable injector (4) according to Claim 1, **characterized in that** the maximum cross-sectional surface area of the housing (200) and of the piston-actuating ram (60) oriented perpendicular to the longitudinal direction (5) corresponds to the envelope contours of the surfaces of the end plate (210) and of the guide arms (78) engaging around them in some areas.

5. Disposable injector (4) according to Claim 1, **characterized in that** the guide arms (78) each have an angled collar (79).

## Revendications

1. Injecteur à usage unique (4) comprenant une unité cylindre-piston (100) pouvant être remplie au moins temporairement d'une substance active, comprenant un boîtier (200), dans lequel est disposé un accumulateur d'énergie à ressort (50) mécanique précontraint et pouvant être libéré et comprenant au moins un poinçon d'actionnement du piston (60) positionné entre l'accumulateur d'énergie à ressort (50) et le piston (111) de l'unité cylindre-piston (100),
- le poinçon d'actionnement du piston (60) présentant deux crochets de traction (78) qui viennent en prise en partie autour du boîtier (200),
- le poinçon d'actionnement du piston (60) pouvant être séparé du boîtier (200) par la détente de l'accumulateur d'énergie à ressort (50),
**caractérisé en ce que**
le boîtier (200) ainsi que le poinçon d'actionnement du piston (60) sont des pièces en tôle à parois minces.

2. Injecteur à usage unique (4) selon la revendication 1,
**caractérisé en ce**
**qu'**au moins le boîtier (200) comprend une plaque frontale (210) munie de fentes (213).

3. Injecteur à usage unique (4) selon la revendication 1,
**caractérisé en ce que**
le boîtier (200) est entouré par un boîtier d'enceinte (270) ayant une section transversale intérieure constante sur sa longueur.

4. Injecteur à usage unique (4) selon la revendication 1,
**caractérisé en ce que**
la surface maximale en section transversale du boîtier (200) et du poinçon d'actionnement du piston (60), orientée perpendiculairement à la direction longitudinale (5), correspond aux contours d'enveloppe des surfaces de la plaque frontale (210) et des branches de guidage (78) venant en prise en partie autour d'elle.

5. Injecteur à usage unique (4) selon la revendication 1,
**caractérisé en ce que**
les branches de guidage (78) présentent à chaque fois un engagement périphérique coudé (79).
